(19) **Europäisches Patentamt**

**European Patent Office**

**Office européen des brevets**

(11) **EP 1 394 181 A1**

(12) **EUROPEAN PATENT APPLICATION**

(43) Date of publication:
**03.03.2004 Bulletin 2004/10**

(51) Int Cl.⁷: **C07K 14/705**

(21) Application number: **02019592.1**

(22) Date of filing: **02.09.2002**

(84) Designated Contracting States:
**AT BE BG CH CY CZ DE DK EE ES FI FR GB GR
IE IT LI LU MC NL PT SE SK TR**
Designated Extension States:
**AL LT LV MK RO SI**

(71) Applicant: **Boehringer Ingelheim International
GmbH
55216 Ingelheim (DE)**

(72) Inventors:
• **Paster, Wolfgang
4153 Peilstein (AT)**

• **Kalat, Milena
1230 Wien (AT)**
• **Kuepcue, Zaruhi
1140 Wien (AT)**
• **Schweighoffer, Tamas
1180 Wien (AT)**
• **Zehetner, Margit
1120 Wien (AT)**

(74) Representative: **Hammann, Heinz, Dr. et al
c/o Boehringer Ingelheim GmbH,
Postfach 200
55216 Ingelheim am Rhein (DE)**

(54) **DNA vaccine**

(57) A DNA vaccine containing, as the active ingredient, a recombinant DNA molecule containing a leader sequence encoding a signal peptide, a sequence encoding one or more antigens, e.g. tumor antigens, a sequence encoding the α3 region of an HLA heavy chain and a sequence encoding a transmembrane region.

**Description**

The present invention is in the field of immunotherapy, in particular in the field of DNA vaccination.

The advent of DNA vaccination during the last decade opened an exciting new era of vaccine research. Although there have been sporadic reports of in vivo transfection already in the 1950s and 1960s (1-3) the potential of these discoveries was largely disregarded until recently. It was only in the early 1990's when two papers showing direct in vivo gene delivery were published. Wolff (4) and Yang (5) with their colleagues showed expression and prolonged biological activity of a reporter gene in murine skeletal muscle, while Tang et al. first described that a specific immune response is initiated towards the gene products (6).

In the following years a series of articles (7-9) demonstrated that plasmid DNA is able to induce potent humoral and cellular immune responses against the encoded antigen in laboratory animals. Genetic immunization was shown to induce protective immunity against bacterial and parasitic infections (10), and also seemed to offer new perspectives for the treatment of cancer and even allergy. However, limited efficiency of DNA-based vaccines still precludes their routine use in human prophylaxis and therapy. Vector structures as well as delivery modalities are thus continuously being modified and optimized. In contrast, relatively little work has been invested into understanding the role which protein structure itself plays for the immunogenicity of embedded antigenic epitopes. For example, subcellular localization, speed and mode of degradation, or post-translational epitope modifications (11, 12) can all influence the dominance of a given antigen. Notably, several laboratories have confirmed that shuttling the class 1 restricted antigenic epitopes directly into the endoplasmic reticulum results in enhanced levels of specific CD8+ T cells (13, 14).

It was an object of the invention to better understand the structural requirements of antigens in order to provide a novel efficient DNA vaccine.

To solve the problem underlying the invention, the potent H-2Kd restricted Cw3 response, first described by Maryanski and coworkers (15), was chosen as a model system to compare and optimize various vaccination strategies. The authors have immunized DBA/2 mice with the heavy chain of the human HLA Cw3 molecule, stably expressed by MHC class I matched transfectants, which provoked robust CD8+ T cell responses. All of the responder T cell clones recognized the dominant epitope (HLACw3.170-179 in its original designation; RYLK for short) presented in MHC H-2Kd context, and showed exclusive usage of the Vβ10 segment in their TCR β chains. Thus RYLK-specific CTLs had the unique phenotype of CD8+CD62L-TCRVB10+ (16, 17). Later it has been established that 97% of RYLK-H2-Kd tetramer sorted cells from Cw3 immunized animals were VB10+ (18). In initial experiments, high-level CTL responses could be elicited in this model system by genetic immunization using the recently developed technique of "electrovaccination" (19). Here the administration of a buffered plasmid DNA solution followed by electroporation at the site of the injection, results in high levels of antigen expression, which in turn provokes massive humoral and / or cellular immune responses. Due to the high level of the Cw3 response, these RYLK-specific cells could be exactly quantified in small samples of peripheral blood by flow cytometry.

In initial experiments of the present invention it was found that plasmid constructs harboring the full-length Cw3 coding sequence reliably induced high-level specific CD8+ T cell responses upon electrovaccination. In contrast to that, it was surprisingly found that plasmids in which the antigenic epitope was engineered after leader sequences that promote ER delivery, provoked only insignificant responses. Nevertheless, the same constructs triggered substantial IFNγ release from epitope-specific CTL lines in vitro upon transfection into H-2Kd+ presenting cells.

In order to find out the reasons for this discrepancy, the inventors conducted broad genetic screens, using the unique combination of a potent plasmid-based immunization protocol together with the fast and accurate detection of specific in vivo responses. In a trans-complementation experiment the participation of trans-acting helper epitopes was ruled out. Systematically altered variants of the full-length Cw3 molecule were then constructed and tested both in vivo and in vitro, which identified the membrane-anchored α3-domain as a crucial determinant of in vivo immunogenicity. Based on these data, new, progressively refined minimal constructs were engineered. In vivo responses elicited by this construct were even higher than those by the full-length molecule. As a proof of concept a melanoma tumor antigen epitope was incorporated into such a high-performer minimal construct. Pronounced protective effects were found in the B16 mouse melanoma model when mice were electrovaccinated with this plasmid.

Thus, the present invention relates to a recombinant DNA molecule containing a sequence selected from

5'-L-(AG)$n$-A3-TM- 3',
5'-L-A3.1-(AG)$n$-A3.2-TM- 3', and
5'-L-A3-(AG)$n$-TM-3', wherein

L denotes a leader sequence encoding a signal peptide that ensures delivery of the expressed molecule into the endoplasmic reticulum (ER) and cleavage of the signal peptide from the molecule AG denotes a sequence encoding an antigen or an antigen fragment or an antigenic peptide;

A3 denotes a sequence encoding the α3 region, or a section thereof, of an HLA heavy chain;

TM denotes a sequence encoding a transmembrane region, or a section thereof, of a membrane spanning protein,

and wherein $n \geq 1$.

In particular, the present invention relates to a vaccine comprising, as the active ingredient, the above-defined DNA molecule.

The leader sequence L comprises or is derived from an export signal sequence encoding a leader peptide that targets a newly synthesized protein to the endoplasmic reticulum of eukaryotic cells. Signal peptide sequences in general contain a hydrophobic core region, but, despite this, they show great variation in both overall length and amino acid sequence. In the present invention, any sequence L (leader or signal sequence) can be used that ensures that the (poly)peptide expressed from the DNA molecule is delivered to the ER of the cell into which the DNA molecule is introduced. Also, it is important that the leader peptide is cleaved from the expressed product.

The leader sequence L may comprise or may be derived from a eukaryotic protein, e.g. from type transmembrane proteins, e.g. CD4, CD8, membrane-bound BCR (B cell receptor; Maddon et al, 1985). Other suitable leader sequences can be obtained from secreted proteins, e.g. immunoglobulins (IgG heavy chain, Ig kappa light chain), SEAP (secreted alkaline phosphatase; Migaki et al, 1995). Preferably, the leader sequence originates from an HLA molecule, e.g. HLA-A*0201, HLA-Cw3.

Alternatively, the leader sequence L may comprise or be derived from a viral protein, e.g. from adenovirus E3 19K protein (13), influenza HA (hemagglutinin protein; Yang et al., 1996), HBsAg (hepatitis B virus surface antigen protein; ref.19).

In order for the leader peptide to be correctly cleaved, the hydrophobic leader signal must be followed by small and neutral residues for correct cleavage by the signal peptidase (31), therefore, it is important to consider the context of the leader sequence with the subsequent sequence element. Usually, the size of the leader peptide encoded by L is in the range of approximately 15 - 40 amino acids (Hanke et al., 1999), but it may be shorter as long as it fulfills its function to deliver the expressed product to the ER.

Depending on the application of the vaccine, i.e. against what disease immunogenicity is to be elicited, the antigen sequence AG encodes a (poly)peptide comprising or being derived from a protein of a viral or bacterial pathogen, in particular a protein that does not exhibit a high mutation rate, e.g. the published examples HPV16/17 (Human Papilloma Virus; Feltkamp et al., 1995), Hepatitis B Virus Core Antigen (Vitiello et al., 1995), Plasmodium Bergheii (Widmann et al., 1992.), Influenza virus hemagglutinin (Yang et al., 1996), Hepatitis C Virus E2 (Zucchelli et al, 2000) or Ebola glycoprotein (Sullivan et al., 2000). Bacterial proteins, such as Yersinia Yop proteins (Leong at al., 1990) or various Staphylococcus proteins (Etz at al., 2002) are also suitable.

In an embodiment of the invention, the antigen sequence AG encodes a tumor antigen or a protein fragment or peptide derived from it. A growing number of tumor antigens are being identified, examples include CEA (Hodge, 1996), gp100 (Kawakami et al., 1994), TRP-1 and TRP-2 (Wang et al., 1996) or CLCA2 (Konopitzky et al., 2002). Other examples are reviewed in Robbins and Kawakami, 1996, and in Schweighoffer, 1997. In this embodiment, the vaccine of the invention is a tumor vaccine.

In another embodiment, AG encodes an autoantigen or a protein fragment or peptide derived from it. Autoantigens are proteins causing autoimmune diseases, e.g. retinal soluble antigen (Boisgerault et al., 1996), MBP (myelin basic protein), PLP (proteolipid protein), MOG (myelin oligodendrocyte glycoprotein) and MAG (myelin associated glycoprotein; all reviewed by Steinman, 1996)

Instead of using an AG sequence that encodes a single antigen or an antigenic protein fragment or peptide (in this case, n=1), the AG sequence may encode a series of identical or different peptides, which represent, upon expression, a so-called "string-of-bead" antigen (Hanke et al., 1999). In this case, n may have a range, depending on the size of the distinct peptides, from 2 to approximately 30.

The size of the antigen region AG can vary within a wide range, e.g. in the case of a single antigen from 8 amino acids to full length proteins of 800 amino acids, while the preferred size of string-of-bead antigens is up to 400 amino acids. In the experiments of the present invention, AG sequences encoding antigens of 9 to 12 amino acids have proven to elicit a T cell response.

With regard to A3, $\alpha 3$ regions are structurally well-defined sections of all MHC and HLA heavy chains; for details see Björkman et al., 1987. For the present invention, the $\alpha 3$ sequence may be derived from any HLA Class I gene, e.g. from HLA-A*0201, HLA-Cw3 or from the so-called non-classical HLA genes, e.g. HLA-E, HLA-G, CD1 family (annotated, aligned and referenced sequences of all these genes are known in the art, e.g. deposited in the IMGT database, publicly available under http://imgt.cines.fr:8104/).

In the case of HLA-Cw3, the size of the natural $\alpha 3$ sequence is approximately 90 amino acids. Sequences A3 in the DNA constructs of the present invention preferably encode the full-length $\alpha 3$ domain; e.g. the full-length HLA-Cw3 domain, but the A3 sequence may also encode an $\alpha 3$ element shorter than 90 amino acids.

In the case that both the leader sequence L and the A3 sequence are derived from an HLA molecule, the sequences may be from the same or from different HLA molecules.

In an embodiment of the invention, the A3 sequence may be interrupted by the antigen sequence AG, i.e. the AG sequence is inserted into the A3 region, creating an A3.1 and an A3.2 region. According to the results obtained in the experiments of the invention, the site of insertion and thus the length of A3 partial sequences on either side of the inserted AG sequence do not appear to be critical.

The TM (also termed TM/IC for transmembrane intracytoplasmic domain) sequence can be derived from any protein having a membrane spanning region, e.g. the proteins mentioned above in the context of the leader sequence, e.g. CD4, CD8, membrane-bound BCR (B cell receptor), immunoglobulins (IgG heavy chain, Ig kappa light chain), SEAP. In a preferred embodiment, the TM region is from a human class I HLA heavy chain or non-classical HLA (see above), in particular from natural or mutagenized human HLA-A, HLA-B or HLA-C alleles, e.g. HLA-A*0201, HLA-Cw3. In the present examples TM sequences from class I HLA molecules have been successfully employed. TM with similar functional characteristics derived from any transmembrane protein may be used as well; to confirm the suitability of other TM regions, they can be tested empirically in series of experiments in a given sequence context.

The minimal size of the TM region is such that it encodes the region that is required for membrane anchorage and correct sorting into the cellular compartments, i.e. it encodes, as exemplified in the experiments of the invention, approximately 16 amino acids. However, extension of the TM sequence by a sequence encoding cytoplasmatic regions of the transmembrane protein is uncritical.

The TM sequence may be extended at either the 5' or the 3'end, e.g. by a sequence encoding a reporter (marker) gene, e.g. the Green Fluorescent protein (GFP) and its mutants, luciferase, HSV thymidine kinase.

Alternatively to a reporter gene sequence short peptide tags (His, FLAG, FLU) may be attached to the TM region. Such extensions are useful for testing the constructs for correct expression and processing. Fluorescent anitbodies for reaction with the tagged expression products are commercially available.

In the embodiment of the invention in which the TM sequence is extended by a tag, the (polypeptides) expressed from the DNA molecules of the invention are useful as surrogate markers. For example, in a therapeutic approach, efficiency of gene expression can be monitored using such tags.

If the TM sequence is derived from an HLA molecule, the HLA molecule may be the same one as the one from which the A3 sequence and the leader sequence are derived, but constructs in which two or all three sequences are from different molecules may also be used. In a preferred embodiment, the L, the A3 and the TM sequence are all derived from HLA-Cw3.

The sequences L, AG, A3 and TM are concatenated with one another, either directly or such that two or all of the expressed sequences L, AG, A3 and TM are coupled by a single amino acid residue or short stretches (2-15) of amino acids. In terms of their order, it is important that L is at the 5' and TM at the 3'end of the DNA molecule. Otherwise, the order of the sequence is uncritical, e.g. A3 may be present in front of AG or in reverse order, or A3 may be interrupted by AG. If AG encodes multiple antigenic peptides, A3 may be inserted in AG sequence, between two sequences encoding distinct peptides.

Overall, the size of the recombinant DNA molecule may vary within a wide range, e.g. encoding a (poly)peptide of approximately 150 - 1,500 amino acids.

For an intended immunization, the optimal sequence of the DNA construct may be determined empirically, according to the principle as described in the Examples. To this end, a panel of antigens is selected in view of the intended therapeutic application, e.g. a tumor antigen or an antigen derived from a pathogen (or fragments thereof). These antigen candidate molecules are combined with preselected candidate sequence elements L, A3 and TM that differ in type and/or size, and the obtained constructs are screened, in a first step, for their ability to be appropriately expressed in vitro, e.g. using the above-described marker-tagged constructs. In a second step, the constructs are testes as to whether they are able to elicit a T cell response, e.g. by determining CD8$^+$ T cell expansion in a suitable animal model system. All sequence elements may be either identical to the naturally occuring sequence or they may be mutagenized.

For preselecting an antigen, it is useful to first determine the immunogenicity of a candidate antigen *per se*, i.e. whether the antigen, or a fragment or peptide derived therefrom, is able to trigger a cellular and/or humoral immune response. This can be tested by methods well known in the art, e.g. as described in Current Protocols in Immunology, Chapter 3, or by Blomberg and Ulfstedt, 1993. The cellular immune response can also be determined by detection of a "delayed-type hypersensitivity" (DTH) reaction in immunized animals (Grohman et al., 1995; Puccetti et al., 1994). Suitable methods for determining the humoral immune response are standard immunoassays (ELISAs) or Western Blots, such methods are also described in laboratory manuals like Current Protocols in Immunology, Chapter 3.

Furthermore the invention relates to a vector carrying the DNA molecule such that it is expressed in the cells of the individual to be treated.

In this embodiment, the recombinant DNA molecule of the invention is in the form of a vector that carries regulatory sequences required for expression of the functional product defined above in mammalian cells, in particular in human cells, most importantly a promoter sequence, e.g. the CMV or SV40 (Takahashi et al, 1986) and RSV or Efα (Mizushima, 1990) promotors; and terminator sequences (e.g. bovine growth hormone or SV40 virus derived polyA). The recombinant DNA vector can be prepared synthetically, for example as shown by Sykes, 1999.

In a preferred embodiment, the complete recombinant DNA molecule is in the form of a plasmid that is capable of self-replication in suitable bacteria, e.g. in E.coli. In this case the plasmid carries, in addition to the recombinant DNA molecule defined above that encodes the functional sequence, a bacterial origin of replication and a selectable marker, such as neomycinkanamycin phosphotransferase or the supF gene. Generally, in view of therapeutic application, construction and production of such plasmids should follow the "Points to Consider on Plasmid DNA Vaccines for Preventive Infectious Disease Indications" guideline issued by the FDA CBER (Center for Biologics Evaluation and Research).

In a further embodiment, the present invention relates to a pharmaceutical composition that contains, as the active ingredient, a plasmid carrying the DNA molecule of the invention. This pharmaceutical composition is a DNA vaccine.

The DNA molecule of the invention can be used in a variety of vaccine formulations based on standard carriers that are suitable for human use, e.g. 150mM NaCI, Ringer's solution, dextrose buffers. The person skilled in the art will be able to select further carriers and auxiliary substances, e.g. balanced salt solutions, which are available in the art (see e.g. Remington's Pharmaceutical Sciences, 2000, and the CBER guidelines, see above).

The DNA molecules of the invention can be administered to an individual in any way that is known for application of DNA in gene therapy applications, either directly or as part of a recombinant virus or bacterium.

Examples of in vivo administration are the direct injection of the plasmid (injection of so-called "naked" DNA), either by intramuscular route or using a gene gun, which has been shown to lead to the formation of CTLs against tumor antigens. Examples of recombinant organisms are vaccinia virus, adenovirus or Listeria monocytogenes (a summary was provided by Coulie, 1997). Moreover, synthetic carriers for nucleic acids such as cationic lipids, microspheres, micropellets or liposomes may be used for in vivo administration. Preferably, administration is combined with physical methods, e.g. electroporation " (19), as described in the Examples.

An example of ex vivo administration is the transfection of dendritic cells as described by Tuting, 1997, or other APCs which are used as a cellular cancer vaccine.

Administration the vaccine of the invention can be combined with protein-type adjuvants, e.g. immunostimulatory polypeptides, in particular cytokines like Interleukin 2 (IL-2) or GM-CSF in a dosage of approximately 1000 units. Further examples for cytokines are IL-4, IL-12, IFN-$\alpha$, IFN-$\beta$, IFN-$\gamma$, IFN-$\omega$, TNF-$\alpha$, and combinations thereof, e.g. IL-2 + IFN-$\gamma$, IL-2 + IL-4, IL-2 + TNF-$\alpha$ or TNF-$\alpha$ + IFN-$\gamma$.

The cytokine (combination) may be administered separately, in the form of a protein, or in the form of a plasmid carrying the sequence encoding it. In that case, the plasmid is preferably administered, in a suitable carrier as described above for the plasmid of the invention, by separate injection.

The vaccine of the invention may also contain conventional adjuvants which enhance the immune response, e.g. BCG (Bacillus Calmette Guerin) and alumn. Usefulness of such adjuvants in combination with a given construct can be tested by administering the DNA construct of the invention with or without adjuvants and determining the immune response in animal experiments. Generally, due to the efficiency of the constructs of the invention, addition of such adjuvants is not required, in particular in the case that the plasmid is administered by electrovaccination.

The experiments of the present invention have proven the advantages of the vaccine of the invention.

In summary, the experiments of the invention have been conducted as follows: Systematically altered variants of the Cw3 molecule were tested both in vivo and in vitro in order to determine the structural parts that are responsible for a potent immune response. In complementation experiments, the participation of trans-acting helper epitopes was ruled out. Successive C-terminal truncations, human/mouse domain swap variants and subdomain modifications defined the $\alpha$3 region of the HLA heavy chain and membrane anchoring as critical elements. Based on these data, refined minimal constructs were engineered that triggered very high in vivo responses. The most advanced variant consisted only of an adenoviral leader, antigenic epitope, $\alpha$3 domain and 16 AA of the TM domain. When a tumor antigen epitope was incorporated into one of these high-performer minimal constructs, protection against melanoma metastases was attained upon vaccination. Thus, structural elements of the antigen can dominantly influence immunogenicity in vivo. These elements can also markedly improve immunogenicity of unrelated antigens, and may form the basis of a new generation of DNA vaccines.

By using an electrovaccination protocol for immunization, high level CTL responses could be elicited. The unusually tight correlation between the phenotype of the responder T cell population and the immunogen in DBA/2 mice immunized with P815-Cw3 cells has been described (17). All of the responding CD8+ T cells directed against a single immunodominant 10mer epitope (the RYLK epitope) share a unique V$\beta$10+ phenotype. This enables their exact enumeration in small samples of the peripheral blood by flow cytometry.

In initial experiments a dramatic expansion of HLA-Cw3 specific VB10+CD8+ T cells in electrovaccinated DBA2 mice could be shown. Peak responses with more than 17% specific CTLs in the peripheral blood were reached at day 13 post electrovaccination. This can be taken as striking proof of the present concept for the DNA vaccination approach on the HLA-Cw3 system. The powerful expansion of CTLs was achieved by an extremely simplified, cell-free immunization system using a minimalist vector with low CpG content and no additional adjuvant.

Surprisingly, the experiments showed considerable differences between in vivo an in vitro immunogenicity of several constructs. Some minimal constructs did turn cells into CTL targets in vitro but were unable to elicit a T cell response in vivo. With this powerful and clearly defined system at hand, the molecular basis of this pronounced CTL response was investigated. Based on the structurally well characterized full length HLA-Cw3 molecule numerous variants of the molecule were created and all tested in vivo.

It has been described that ER targeting improves immunogenicity especially of subdominant class I epitopes (13, 32). Surprisingly and in sharp contrast to the full length construct #513, ER-targeted epitope-only constructs failed to stimulate CD8+ T cells in vivo. This is clearly not a matter of inefficient expression or liberation of the epitope as the same constructs did transform cells into targets for RYLK specific CTLs in vitro. These findings indicate the importance of a correct structural environment of the antigenic RYLK epitope for the induction of potent CTL responses in vivo. Apparently the antigenic epitope encoded by the minimal constructs are expressed, processed, and presented on the cell surface efficiently in vitro, but not in vivo. Professional APCs haven been shown to play a major role during DNA vaccination (33, 34). It could well be that the highly active antigen processing machinery of these cells effectively destroys the shorter RYLK epitope-only antigens while longer constructs have a prolonged existence as antigen depots.

Short minimal epitope constructs apparently are missing important structural elements. Besides their obvious structural function these elements could also act in trans, providing T cell help. There are indeed reports on trans acting helper epitopes, processed and presented by the same APC as the antigenic epitope itself (25, 26). The participation of trans acting helper epitopes was directly ruled out by a trans complementation experiment. None of the constructs provided could supplement for missing structural parts of minimal construct #540 in trans. Not even plasmid #529, despite being HLA-Cw3 full length only with the immunogenic epitope replaced by the RYLE homologue of MHC H2-K$^d$. These results indicate that all structural elements necessary have to be located on the same stretch of amino acids (in cis) as the antigenic RYLK epitope. Possible cis structural requirements for enhanced in vivo antigenicity could be correct folding for efficient processing, organelle specific trafficking, membrane retention, or interaction of various domains with different molecules.

Dissection of the structural requirements was begun by in vivo testing of domain-wise C-terminal truncation mutants. C-terminal truncation variants showed a two step pattern in their loss of immunogenicity: the first major loss occurred upon truncation of the TM/IC domain, when $\alpha$3 was truncated additionally, the percentage of VB10+CD8+ T cells dropped down to background levels.

To further characterize the contribution of these two regions, mutant plasmid variants with internal truncations of the $\alpha$2/$\alpha$3 domains were designed. Only minor losses in immunogenicity could be linked to the truncation of $\alpha$3 in vivo, whereas in vitro these constructs performed equally well. As all these constructs where membrane anchored, the endogenous HLA-Cw3 TM/IC domain was considered the most likely determinant of the HLA-Cw3 molecule for high in vivo responses against the RYLK epitope.

Surprisingly, even additional stepwise C-terminal truncations of TM/IC showed no adverse effect until complete removal of the domain. Plasmid #709, with a rudimentary TM anchor of 16 AA, produced a response as high as the positive control #513. These findings clearly indicate that membrane anchoring, most likely in an organelle specific manner, without intracellular domains is essential.

That organelle specificity plays an important role was confirmed by TM/IC domain replacement variants. Plasmids with transmembrane domains of HLA H2-IA (#741) and CD47 (#740) and with sequences encoding for post-translational GPI-link modification failed to stimulate considerable amounts of CTLs. For the GPI-linked constructs it is obvious that exclusive sorting to the plasma membrane (35) accompanied with different lateral mobility removes much of the antigen from the processing / presentation machinery.

Summing up all these data, a series of progressively refined minimal constructs was designed. Adding the endogenous TM/IC domain to the minimal epitope construct #540, which reportedly did not stimulate CD8+ T-cells in vivo, did boost in vivo immunogenicity significantly. As CTL levels still were not comparable to those elicited by full length HLA-Cw3, the $\alpha$3 domain, identified as the second most important structural part, was additionally reinserted. By doing so, a construct even superior to plasmid #513 could be obtained. Thus, besides the importance of TM/IC, also $\alpha$3 apparently plays a role in efficient processing of HLA-Cw3. This finding comes as a surprise since the $\alpha$3 domain had undoubtedly been shown to be of only minor importance before. On the other hand, the previous results had been obtained in the more complex full-length environment with the domains $\alpha$1 and $\alpha$2 still on the molecule. In contrast to a short and minimalist construct, these two domains in the multi-domain molecule could compensate for the missing $\alpha$3 domain. Thereby potential truncation effects got masked.

In conclusion, in the experiments of the invention short ER-targeted epitope-only constructs did not work in vivo. An endogenous minimal TM anchor and the $\alpha$3 domain could restore antigenicity completely. An explanation for this structure dependence could be that efficient Cw3 constructs behave similarly to endogenous class I molecules. A small fraction of class I molecules has been shown to recirculate from the plasma membrane to endosomal compartments (36). It can now be hypothesized that this happens with the Cw3 molecule in APCs, thereby providing the cell with a long-term antigen reservoir. Minimal constructs do not have access to this pathway and are therefore highly prone to rapid proteasomal degradation. The additional requirement for the $\alpha$3 domain implicates interaction with receptor structures since this Ig-fold domain is contacted, among others, by CD8 and the antigen loading machinery.

Maybe special characteristics of the Cw3 α3 domain allow atypical interactions with intracellular factors or receptor structures on professional (p)APCs when present on the plasma membrane of transfected "factory cells". Such contact with pAPCs could finally lead to transfer of membrane vesicles containing the antigen (37).

While many of the previous studies focussed on optimization of vector backbone and delivery strategy, the experiments of the invention have successfully shown that protein structure of the antigen itself is important. Often reported to be relatively inefficient in inducing immune responses, DNA vaccination using the system of the invention stimulated tremendous CD8+ T cell responses. This was accomplished by using a minimalist vector with low CpG content and without using adjuvant for injection. Having obtained promising data in the B16 mouse melanoma model using the TRP-2 tumor antigen in a Cw3 minimal construct, the structure-function relationship deduced on the basis of the HLA-Cw3 system provides the basis for exploiting the findings obtained in the HLA-Cw3 system for vaccine development. The vaccine of the invention is superior to previous DNA-based vaccines used in the B16 model and it is to be expected that the DNA vaccines of the invention will boost immunogenicity of inefficient CTL epitopes.

Brief description of the figures:

Figure 1:
Anti-Cw3 responses in DBA/2 mice show pronounced differences depending on the context of the dominant epitope.

Figure 2:
Minimal constructs that do not induce expansion of CD8+ T cells in vivo still do stimulate HLA-Cw3 specific CD8$^+$ T-cells in vitro.

Figure 3:
Inefficient in vivo performance of a minimal construct can not be complemented for in trans.

Figure 4:
C-terminal truncation of the full-length HLA-Cw3 constructs narrows down the region essential for effective in vivo responses.

Figure 5:
Membrane anchoring is crucial for effective in vivo responses while the α3 domain is dispensable.

Figure 6:
Alterations in transmembrane domain have profound effects on antigenicity of HLA-Cw3 in vivo.

Figure 7:
Newly defined, efficient minimal constructs can be utilized as carrier for an unrelated, weakly immunogenic CTL epitope.

In the Examples, if not otherwise stated, the following abbreviations and materials and methods were used:

E3       Adenovirus E3 19k protein leader sequence
EGFP     enhanced green fluorescent protein;
ER       endoplasmic reticulum
IC       intracytoplasmic domain
RYLK     H-2Kd-restricted Cw3 epitope RYLKNGKETL;
RYLE     MHC-H2 encoded homologue of RYLELGNETL
SVYD     mTRP-2-derived epitope SVYDFFVWL
TM       transmembrane portion

Mice

Eight week-old female DBA/2 mice were purchased from Harlan, Germany and held under specified pathogen-free conditions. Mice were acclimated one week before the start of the study. All animal work was performed in full compliance to institutional and legal guidelines.

Cell lines

The murine colon carcinoma cell line C-26 was described previously by Rodolfo at al. The murine mastocytoma cell line P815 was obtained from ATCC (Manassas, VA). P815 #123, stably expressing the minimal construct #123, were obtained after P815 cells were transfected with the plasmid #123 by electroporation. Stable clones showing high expression of the fusion protein were derived by chemical selection, FACS sorting and limiting dilution. B16F10, a murine melanoma cell line of C57/BL/6 origin (Fidler et al., 1975) was obtained from the NIH DCTDC Tumor Repository, from which the pigmented, TRP-1,2 expressing and in vivo aggressively growing B16F10M subline was subcultured in our laboratory.

Tumor challenge experiment

For the B16 lung metastasis model, groups of C57/BL6 mice with 10 animals each were electrovaccinated three times with intervals of one week, using 50 µg of plasmids #789 and #790. Both constructs harbor the dominant SVYD epitope derived from the tumor antigen mTRP-2. Control mice (2 groups with 10 animals each) were left untreated. One week after the last vaccination mice were challenged by i.v. injection of $8x10^5$ B16F10M cells in 100 µl D-PBS. The number of cells needed was established by previous titration experiments, and gives reproducibly a lung surface coverage of >80% after three weeks. To avoid aggregation of cells, the suspension was kept on ice until injection. On day 22 post-challenge, mice were sacrificed and lungs were examined for coverage with metastases. Percent of surface covered with metastases was estimated.

Epitopes, plasmids and immunizations

RYLK is the short-hand notation for the (DNA) sequence that encodes the H-2Kd-restricted dominant Cw3-derived epitope RYLKNGKETL; RYLE stands for the MHC-H2 encoded mouse homologue RYLELGNETL. SVYD denotes the dominant H-2Kb-restricted epitope SVYDFFVWL of mTRP-2 expressed by the murine melanoma B16 (20).
The vector pVAX1 (Invitrogen) or its derivatives were used throughout the in vivo immunization protocols. Construction was according to the Food and Drug Administration (FDA USA) document, "Points to Consider on Plasmid DNA Vaccines for Preventive Infectious Disease Indications".
CpG content and, consequently, immunogenicity of the plasmid backbone was therefore lowered (21). Features of the vector allow high-copy number replication in E.coli and high-level transient expression of the protein of interest in most mammalian cells. The vector contains the following elements:

- Human cytomegalovirus immediate-early (CMV) promoter for high-level expression in a wide range of mammalian cells
- Bovine growth hormone (BGH) polyadenylation signal for efficient transcription termination and polyadenylation of mRNA
- Kanamycin resistance gene for selection in E.coli

Sequences not necessary for replication in E.coli or for expression of recombinant protein in mammalian cells were removed to limit DNA sequences with possible homology to the human genome and to minimize the possibility of chromosomal integration. The kanamycin resistance gene was substituted for the ampicillin resistance gene because aminoglycoside antibiotics are less likely to elicit allergic responses in humans. CpG content and, consequently, immunogenicity of the plasmid backbone was therefore lowered. All constructs were designed using the DNA Star sequence analysis software package (DNA Star Inc.).
Starting from single colonies, 5 ml precultures in LB (1% BactoTryptone, 0.5% BactoYeast, 10mM NaCl, 10mM MgS04, pH 7.5 + selective antibiotic) were grown overnight at 37°C. 1 ml preculture was added to 1l LB (+selective antibiotic) and bacteria were grown overnight (max. 16h) at 37°C on a shaker. Bacteria were centrifuged at 5 krpm for 15min and after complete removal of media, bacteria were washed once in PBS. After centrifugation and removal of fluid, bacteria were resuspended completely in 30 ml buffer P1 (QIAGEN 19051) + 100 µg/ml RNase A. After adding 30 ml buffer P2 (QIAGEN 19052) and gentle mixing, cells were lysed for 5 min at room temperature. 30 ml neutralization buffer P3 (QIAGEN 19053) was added and after carefully mixing the lysate was incubated at 4°C for 10 min. The lysate was cleared by centrifugation at 10 krpm for 15 min and subsequent filtering. DNA was precipitated by adding 0.6-1 volumes of Isopropanol and centrifugation at 10 krpm for 30 min at 4°C. Salts were removed by washing the pellet with 70% Ethanol. DNA was dissolved in 4 ml TE buffer (10 mM Tris pH 8.0, 0.5 mM EDTA pH 8.0) and 4.85g CsCl (Boehringer Mannheim 757306) was added. After complete dissolving of CsCl on a 37°C water bath, the mixture was centrifuged quickly to destroy bubbles and filled into VTi65-quickseal tubes (Beckman 342412) with a syringe. The tubes were balanced exactly, sealed and centrifuged in a Beckman L8-60M Ultracentrifuge at 60 krpm for 16 h at 20°C without braking at the end of the run. 200 µl fractions were taken, 0.2 µl samples of which were run on a 0.8 % agarose gel to check for DNA content and RNA contamination. The best fractions (high DNA, low RNA) were pooled and dialyzed overnight at 4°C against MQ H2O in a Slide-A-Lyzer 10K dialysis cassette (PIERCE 66425). DNA retrieved from cassette was quantified and frozen in aliquots.

To verify newly constructed plasmids, DNA sequencing was predominantly used on mini preps. Reactions were done on a 10 µl scale using 1 µl mini prep DNA, 4 µl Sequencing Mix (ABI PRISM BigDye Terminator Cycle Sequencing Mix), 4.5 µl MQ H2O and 0.5 µl 10pM/µl primer (=5 pM per reaction). Sequencing reactions were done on a Perkin Elmer GeneAmp PCR System 9600 Thermocycler with the following conditions: 95°C, 2 min / 30x [95°C, 30sec ; 50°C, 30sec. 72°C, 120sec ] / 72°C, 5min / 4°C, ∞. DNA was precipitated by adding 1 µl LPA (linear polyacrylamide), 2 µl dextran blue (20 mg/ml), 1.5 µl 3 M NaAc and 50 µl ice cold Ethanolabs and centrifugation at 10 krpm for 30 min at 4°C. After complete removal of the supernatant the pellet was washed with 70 % Ethanol. In the form of air-dried pellets products were submitted to the in-house sequencing service.

Throughout the entire work Promega enzymes in the buffer supplied by the manufacturer were used for restriction digests. Usually between 2 µl (for diagnostic digests) and 15 µl (for preparative digests) of miniprep DNA was used. Of higher purity plasmids a maximum of 1 µg was digested. Digests were purified by agarose gel electrophoresis and subsequent gel elution of the fragments of interest. For ligation a molar ratio of vector:insert=1:3 in a reaction volume of 20 µl 1 x T4 Ligation buffer (Boehringer Mannheim 1243292) with 2 µl of T4 DNA-Ligase (BoehringerMannheim 716359; 1 u/µl) and 1 µl of 10 mM rATP was used. Incubation was 4-5 h at RT or if higher efficiency was needed overnight at 14°C. Ligation reactions were transformed by electroporation into E. coli.

Ligation reactions generally were performed in volume less than 20 µl. DNA was precipitated by adding 1 µl LPA, 1.5 µl 3 M NaAc and 60 µl of ice cold Ethanolabs and centrifugation at 14 krpm for 30 min at 4°C. After complete removal of the supernatant the pellet was washed with 70 % Ethanol and air dried. DNA was dissolved in 20 µl MQ H20 and 10 µl were used for electroporation. The remaining half was stored at -20°C as a backup. Electrocompetent bacteria were thawn on ice. Electroporation cuvettes (Equibio ECU-101 50) and DNA were cooled on ice. 40 µl bacteria were mixed with 10 µl DNA avoiding air bubbles and the mixture was carefully filled into the precooled cuvette. One pulse of a Biorad Gene Pulser II with 1.8 kV/20 Ω/25 µF was applied with a time constant higher than 4 ms necessary for efficient DNA uptake. Bacteria were flushed out of the cuvette with 450 µl room temperature SOC (2% BactoTryptone, 0.5% BactoYeast, 10mM NaCl, 10mM MgCl2, 10mM MgSO4 and 20mM glucose) and incubated for 1 h at 37°C on a shaker. 30 and 180 µl of the preculture were plated on each half of LB/AMP plates containing the appropriate selective antibiotic (100 µg/ml for ampicillin and 50 µg/ml for kanamycin). Plates were incubated overnight at 37 °C.

Plasmids were either purified on CsCl gradients or custom manufactured by ELIM Pharmaceuticals (South San Francisco, CA) and contained less than 5 EU/mg endotoxin. Required amounts of plasmids were diluted into 100 µl 20mM HEPES pH7.4 buffer and 50µl each were applied to both quadriceps femoris muscle of mice under Avertin anesthesia. Immunization. was followed immediately by electroporation of the injected area (80V, three pulses of 60ms with repoling) using an Electro Square Porator device (T820; BTX, San Diego CA).

FACS analysis

200µl blood was sampled from the retroorbital sinus using heparinized capillaries and tubes. Lymphocytes were isolated by gradient density centrifugation (Lymphoprep; Nycomed AS). Mononuclear cell yield after micro-Lymphoprep was $3-5\times10^5$ per bleed. Samples from each animal were processed and analyzed individually, without pooling. Normal rabbit immunoglobulin and anti-CD16/32 mAb were added to block unspecific sites first, followed by a mixture of anti-CD62L-FITC, anti-CD8-PE and anti-Vβ10-biotin (all antibodies from B-D Pharmingen). APC- or Cychrome-conjugated streptavidin (both from Pharmingen) were used as second step reagents. Cells were analyzed on a FACSort or B-D LSR cytometer, and more than 300,000 events gated on PBL were collected. Data were analyzed using WinMDI (J.Trotter, Scripps Institute) and percent specific CD8+ cells calculated in two steps: first live, CD8+ cells were electronically gated. Then on a CD62L (x-axis) vs. Vβ10 (y-axis) dot plot displaying these

$$\% \text{ specific cells} = \frac{(CD62L - VB10 +) - \dfrac{(CD62L - VB10 -)\times(CD62L + VB10 +)}{(CD62L + VB10 -)}}{totalCD8 +}$$

gated cell only, four quadrants were set which fined high and low-expressing cells. In order to obtain precise, unbiased values, the low-level spontaneous loss of CD62L was corrected by employing the following equation (22, 23). Background measured on naïve and control-treated mice using this calculation was 0.3±0.1%.

IFNγ-ELISA

C-26 murine colon carcinoma cells were grown overnight in 96-well plates at 15,000 cells per well and transiently transfected with 500 ng DNA per well using the DEAE-Dextran / Chloroquin method (Simmons, 1993). After 3.5 h incubation the transfection mixture was removed from the cells and fresh media was added. The day after transfection 15,000 HLA-Cw3 specific CTLs were added per well. After four days of cocultivation supernatants were analyzed for their IFNγ content by ELISA. For quantification of IFNγ in cell culture supernatants the OPTEIA Mouse IFNγ antibody set (Pharmingen) was used.

Example 1

Not all epitope-expressing constructs which transform cells into CTL targets in vitro do elicit a T cell response in vivo

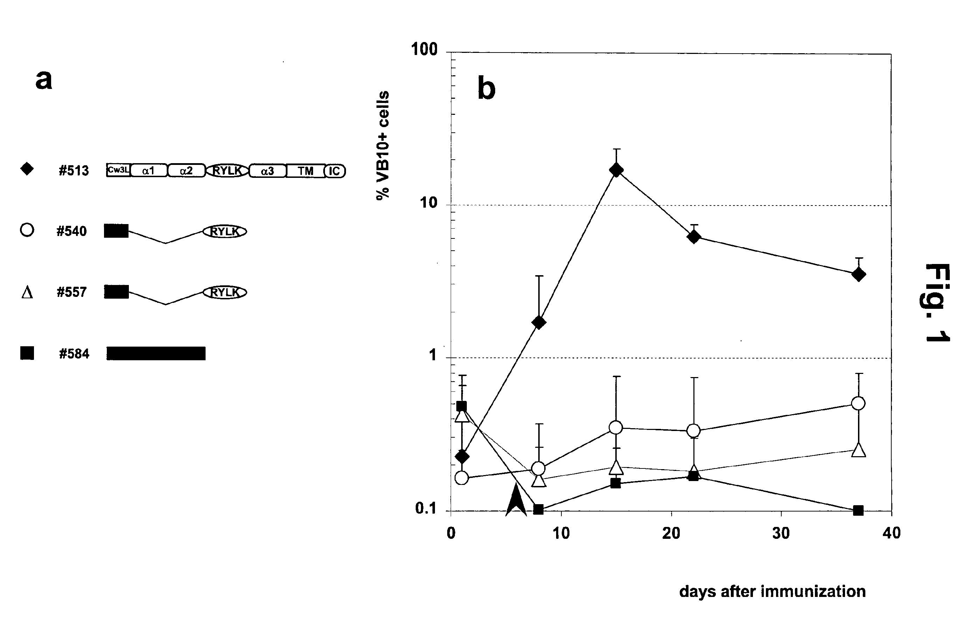

A) In an initial time-course experiment the kinetics of the CD8+ T cell expansion against the dominant H-2Kd restricted HLA-Cw3 epitope was analyzed in vivo. Three different constructs were compared (Figure 1a): plasmid #513 that encodes full length HLA-Cw3 provoked high CD8+ T cell levels already in previous experiments and served as positive control. The two others were minimal constructs where the antigenic RYLK epitope was preceded by leader sequences to ensure efficient translation and ER delivery of the product (24): plasmid #540 contains the Adenovirus E3 19k leader, and plasmid #557 the immunoglobulin light chain leader. Plasmids were electrovaccinated on day 2, and PBMC analyzed by FACS at days 1 (pre-vaccination), 8,15, 22 and 37 (Figure 1b). A steady increase in the percentage of VB10+CD8+ T-cells could be observed with the full-length construct #513. After a peak of more than 17% at day 15, the response slowly decayed. These and other data also suggested that a period of 13 days between electrovaccination and FACS analysis of PBMCs was optimal for the measurement of the peak response, and was used in all other in vivo experiments. Surprisingly the two minimal constructs #540 and #557 did not elicit CD8+ T cell responses above background.

Figure 1:

(a) Representative structures of plasmids used for immunization.

Note that Cw3-derived sequences are depicted with symbols on white background, whereas structural parts of non-Cw3 origin are on black background. Plasmid #513, HLA Cw3 full length. Plasmid #540, RYLK epitope preceded by Adenoviral E3 19k leader. Plasmid #557, RYLK epitope preceded by immunoglobulin light chain leader. Plasmid #584, human growth hormone coding sequence.

(b) Time course of RYLK-specific CD8+ T cell expansion.

Groups of DBA/2 mice with four animals each were electrovaccinated with 50 μg/animal of plasmid #513 (♦), #540 (□), or #557 (△). Control mice (■) received #584. Vaccine administration is indicated by the arrowhead. The day before vaccination and at days 8, 15, 22 and 37 post-vaccination blood samples of 200 μl were taken and lymphocytes were isolated by gradient density centrifugation. Cells were CD8/CD62L/VB10 triple stained. VB10+CD62L- cells among CD8+ lymphocytes were quantified by FACS. Samples were treated individually, without pooling in all experiments. Note that percentage of VB10+ cells is shown on a logarithmic scale.

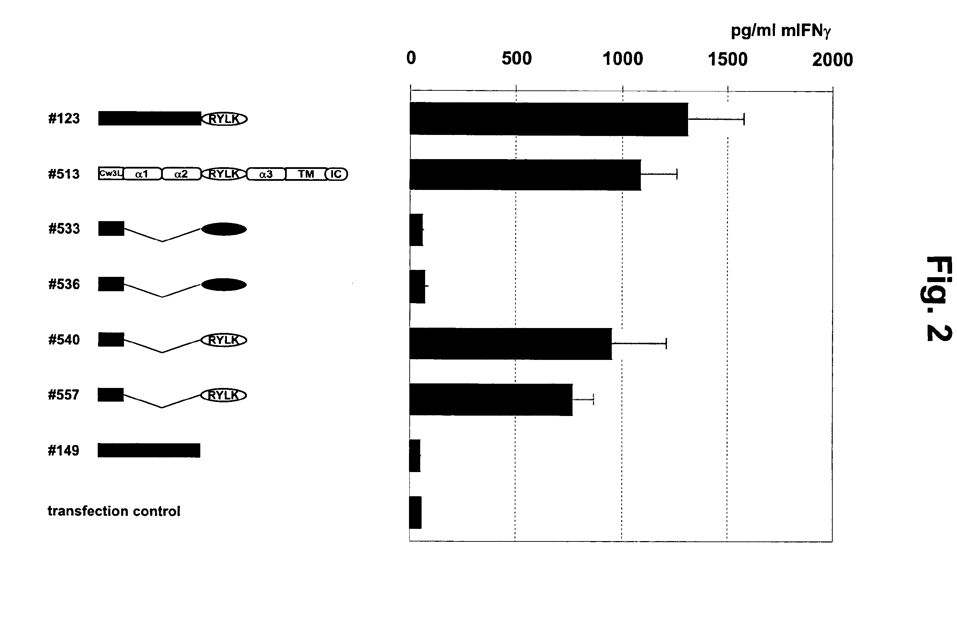

B) To see whether extensive changes in structure would interfere with efficient expression and antigen presentation of minimal constructs #540 and #557 in murine cells, these and other minimal epitope constructs were tested in vitro (Figure 2). C-26 mouse colon carcinoma cells were transiently transfected with plasmid DNA and cocultivated with HLA-Cw3 specific CTL lines. Supernatants were collected after 4 days and their IFNγ content measured by ELISA. Again, plasmid #513 served as positive control. In addition to constructs #540 and #557, plasmid #123, encoding the RYLK epitope fused to the C-terminus of EGFP, was introduced as another minimal construct. Plasmids #533 and #536 are respective homologues of plasmids #557 and #540, except that the RYLK epitope was replaced by the SVYD epitope, derived from the murine B16 tumor antigen TRP-2. Plasmid #149 encoded EGFP only. In contrast to the in vivo findings, the two minimal constructs #540 and #557 are comparable to plasmid #513 in transforming cells into CTL targets in vitro. Also plasmid #123 stimulates strong IFNγ production that is clearly directed against the RYLK 10-mer, as can be seen by comparison to plasmid #149 that provokes only background stimulation of CTLs. As expected, cells transfected with plasmids #533 and #536, which harbor the SVYD epitope, do not stimulate HLA-Cw3 specific CTLs in vitro.

Figure 2:

C-26 mouse colon carcinoma cells were transiently transfected with the indicated plasmids and cocultivated with HLA-Cw3 specific CD8$^+$ T cells. Supernatants were collected after 4 days and their IFN$\gamma$ content measured by ELISA. Representative structures of plasmids used are shown. Note that Cw3-derived sequences are depicted with symbols on white background, whereas structural parts of non-Cw3 origin are on black background. Plasmid #123, RYLK epitope fused to the C-terminus of EGFP. Plasmid #513, HLA Cw3 full length. Plasmids #533 and #536, TRP-2 derived epitope SVYD preceded by by immunoglobulin light chain leader and Adenoviral E3 19k leader, respectively. Plasmid #540, RYLK epitope preceded by Adenoviral E3 19k leader. Plasmid #557, RYLK epitope preceded by immunoglobulin light chain leader. Plasmid #149, EGFP only.

In conclusion, RYLK epitope carrying minimal plasmid constructs that were well expressed and did stimulate HLA-Cw3 specific CTLs in vitro failed to elicit a specific CTL response in vivo upon electrovaccination. Thus, generally the expression of epitope-only minimal constructs is not severely impaired and it is unlikely that simple variations in expression levels caused the dramatic differences between full-length and minimal constructs in vivo. It was therefore reasoned that portions of the full-size Cw3 molecule which lie outside of the dominant epitope must be responsible for this remarkable difference, and performed a genetic complementation experiment.

Example 2

Epitope-only constructs can not be trans-complemented in vivo

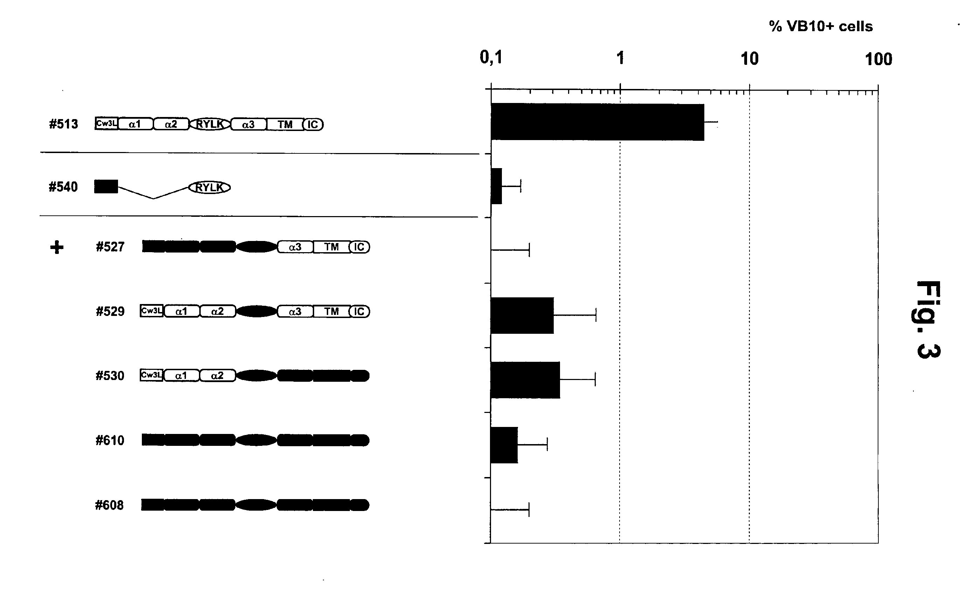

Trans-complementation was used to address possible involvement of helper epitopes (25, 26). Plasmid #540, that harbors a RYLK epitope preceded by the adenoviral E3 leader, induced only low levels of specific CD8+ T cells in vivo (Figure 3). This construct was then coinjected with one of the following chimerical human/mouse plasmid variants: plasmid #527 that encodes $\alpha1/\alpha2$/RYLE from MHC H-2Kd and $\alpha3$/TM from HLA-Cw3; plasmid #529 that encodes full-length HLA-Cw3 except that the epitope is mutated to the RYLE homologue; plasmid #530 that encodes $\alpha1/\alpha2$ from HLA-Cw3 and RYLE/$\alpha3$/TM from MHC H-2Kd; plasmid #610 that encodes full length MHC H-2Kd and plasmid #608 that encodes full length MHC H-2Kb.

Figure 3:

Groups of DBA/2 mice with four animals each were electrovaccinated with 50 $\mu$g/animal of plasmid #540 and the respective complementing plasmid, resulting in a total of 100 $\mu$g DNA. Blood samples were analyzed 13 days after electrovaccination. Plasmid #513, HLA Cw3 full length. Plasmid #540, RYLK epitope preceded by Adenoviral E3 19k leader. Plasmid #527, $\alpha1/\alpha2$/RYLE from MHC H-2Kd and $\alpha3$/TM from HLA-Cw3. Plasmid #529, full-length HLA-Cw3 except the epitope is mutated to the RYLE homologue. Plasmid #530, $\alpha1/\alpha2$ from HLA-Cw3 and RYLE/$\alpha3$/TM from MHC H-2Kd. Plasmid #610, full-length MHC H-2Kd. Plasmid #608, full length MHC H-2Kb.

None of the plasmids injected together with #513 could significantly complement for missing elements in plasmid #540, when compared to the robust Cw3 response induced by the positive control #513. Even plasmid #529 that carries all structural elements of HLA-Cw3 except the antigenic epitope, induced a response only marginally above background.

These data suggest that putative critical structural parts must reside on the very same molecule where also the epitope is present; they must be in some ways physically connected.

Example 3

C-terminal truncations of HLA-Cw3 assign a significant role for the $\alpha3$/TM region in the induction of potent CD8+ T cell responses in vivo

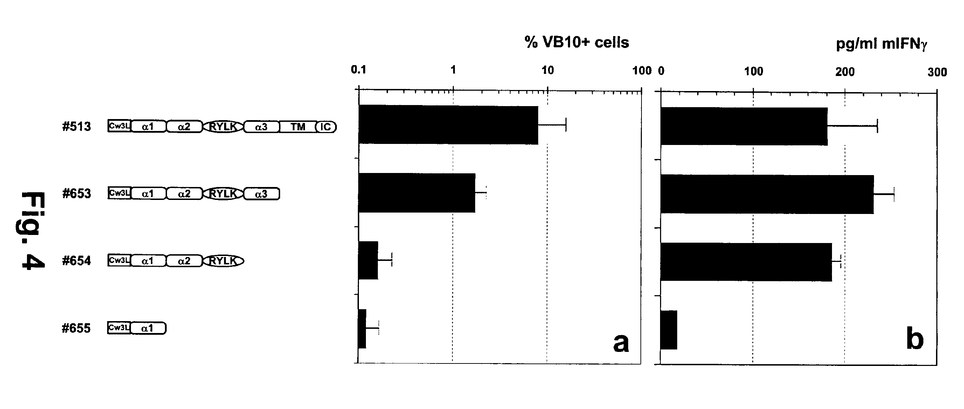

The localization of important structural elements was then started by successive C-terminal truncations of the full-length HLA-Cw3. In plasmid #653 the TM/IC domains were truncated completely, the resulting molecule can thus be considered being soluble. Plasmid #654 encoded a HLA-Cw3 molecule where in addition also the $\alpha3$ domain was deleted. Finally, plasmid #655 only encoded the $\alpha1$ domain, and lacked the RYLK epitope. The truncation mutants together with plasmid #513 were then first tested in vivo (Figure 4a). By truncation of the TM/IC domains (#653) the expansion of VB10+CD8+ T cells was reduced by 80% compared with the positive control. This level dropped to background when the $\alpha3$ domain was deleted additionally (#654). With just the $\alpha1$ domain left, plasmid #655 did not elicit any expansion of VB10+CD8+ T cells.

Also with these plasmids the in vitro and in vivo results were markedly different (Figure 4b). Essentially all constructs carrying the RYLK epitope (#513, #653 and #654) were similarly effective in vitro, regardless of their C-terminal truncations. CTL recognition was lost completely only when the RYLK epitope was missing. These results clearly indicated that structures within the α3/TM domain were key contributors to an efficient in vivo CD8+ T cell response.

Figure 4.

(a) Successive truncation of the transmembrane and the α3 domains results in stepwise loss of antigenicity. Groups of four DBA/2 mice were electrovaccinated with 50 µg/animal of plasmid and blood samples were analyzed 13 days after electrovaccination.
(b) Stimulation of CTLs in vitro depends on the presence of the epitope but is otherwise independent from truncation. C-26 mouse colon carcinoma cells were transiently transfected with the indicated plasmids, cocultivated with HLA-Cw3 specific CD8+ T cells and released IFNγ was measured by ELISA. Plasmid #513, HLA Cw3 full length. Plasmid #653, TM/IC domains truncated completely. Plasmid #654, α3 domain deleted additionally. Plasmid #655, α1 domain only.

Example 4

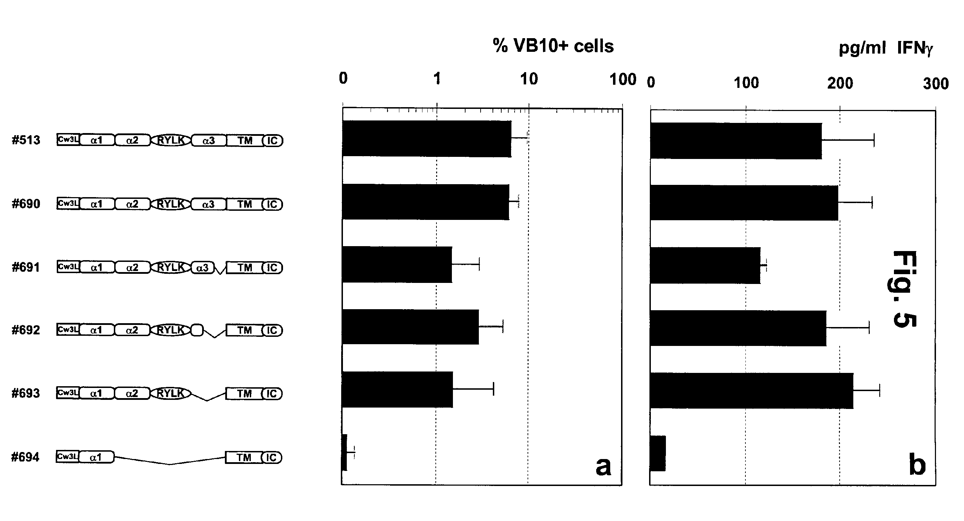

Membrane anchoring is important for high immunogenicity of HLA-Cw3 in vivo, while the α3-domain can be deleted without considerable loss of antigenicity
To further narrow down critical regions, truncations on the C-terminal side of the α3-domain were introduced. The TM/IC domain was then fused to the remaining molecule (Figure 5). Together with plasmid #513 as positive control, these constructs were tested in vivo (Figure 5a). Two of the four resulting constructs contained parts of the α3 domain extending from AA 203 to 268 (#691 ) and from AA 203 to 241 (#692), respectively. In plasmid #693 the α3 domain was completely truncated, and in plasmid #694 the α2 domain was deleted additionally. No substantial losses in the expansion of VB10+CD8+ T cells were observed, even with α3 completely removed from the molecule (plasmid #693). As expected, plasmid #694 that lacks the RYLK epitope, only stimulated background VB10+CD8+ T cell expansion. In vitro these findings could be confirmed (Figure 5b). Again, removal of the α3 domain did not decrease antigenicity of the remaining molecule. These data confirmed that membrane anchoring was an important factor for eliciting strong CD8+ T cell responses in vivo.

Figure 5:

(a) Successive truncations of the α3 domain with the transmembrane domain still fused to the remaining molecule. Groups of four DBA/2 mice were electrovaccinated with 50 µg/animal of plasmid and blood samples were analyzed 13 days after electrovaccination.
(b) Stimulation of CTLs in vitro. C-26 mouse colon carcinoma cells were transiently transfected with the indicated plasmids and cocultivated with HLA-Cw3 specific CD8+ T cells. Released IFNγ was measured by ELISA. Plasmid #513, HLA Cw3 full length. Plasmid #690, full length HLA Cw3 carrying a BstEII restriction site at the α3/TM domain interface. This site was also used for plasmids #691 to #694 for fusion of the endogenous TM domain to the truncated C-terminal part of HLA-Cw3. Plasmid #691, AA 203 to 268 of α3 domain. Plasmid #692, AA 203 to 241 of α3 domain. Plasmid #693, α3 domain completely truncated. Plasmid #694, α2 domain deleted additionally.

Example 5

The endogenous transmembrane domain of HLA-Cw3 is critical for the immunogenicity of RYLK-carrying molecules and can be used to engineer a synthetic minimal construct

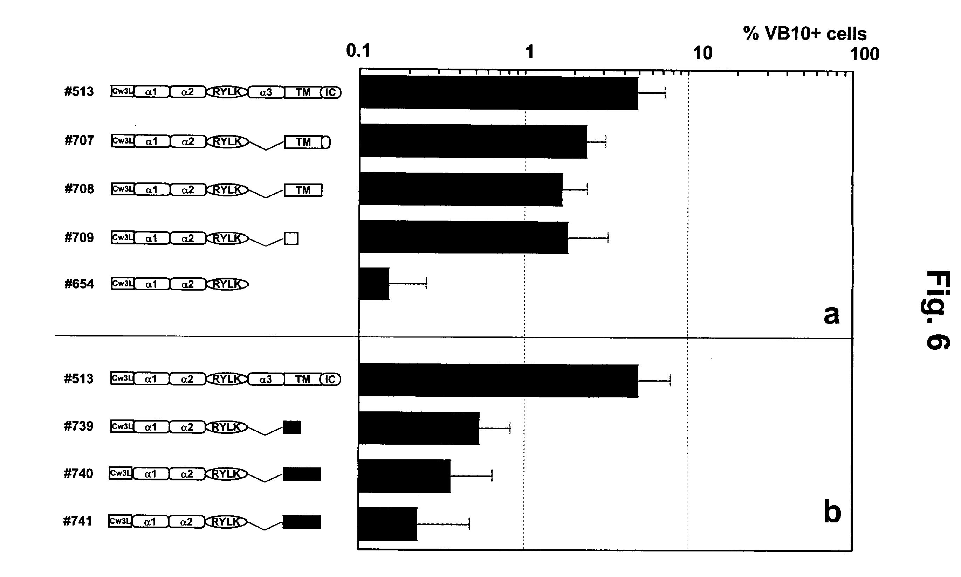

A) Fine mapping of critical regions inside the TM/IC domains was begun with C-terminal truncations in the respective domains. On the basis of plasmid #693 the TM/IC was successively truncated, of which plasmid #707 carries AA 298 to 350, plasmid #708 carries AA 298 to 339 and plasmid #709 carries AA 298 to 314. The final step in this series was the soluble variant #654. The resulting plasmids were tested in vivo. Standard positive control #513 is shown for comparison. (Figure 6a). Dramatic decreases in CD8+ T cell levels were only detected after the TM domain was completely removed. Even plasmid #709, with just 16 AA remaining of the TM domain, induced high-grade C08+ T cell expansions in vivo.

B) To test the significance of compartment-specific TM anchoring, the endogenous TM domain of HLA-Cw3 was exchanged for TM domains of different molecules (Figure 6b). In plasmid #739 sequences encoding post-translational GPI-link modification were fused after the RYLK epitope of the $\alpha$3-deleted #693 instead of the endogenous TM/IC. In two other constructs the endogenous TM domain was exchanged: in #740 for the TM domain of CD47, and in plasmid #741 for the TM domain of H2-IA. Plasmids #739, #740 and #741 showed a significantly decreased capacity of VB10+CD8+ T cell expansion. Nevertheless, responses provoked by these constructs were still higher than by soluble #654. Thus while the rudimentary endogenous TM domain is sufficient for the induction of strong responses in vivo, it can only be partially substituted with foreign variants. Confocal microscopy of an EGFP tagged variant of #709 suggested that this construct retained its membrane anchoring (data not shown).

Figure 6:

(a) A rudimentary endogenous TM domain is sufficient to promote high antigenicity.
Successive truncations of the endogenous TM domain were fused to the first two domains plus RYLK epitope. Plasmid #513, HLA Cw3 full length. TM/IC truncation mutants are based on #693. Plasmid #707, AA 298 to 350. Plasmid #708, AA 298 to 339. Plasmid #709, AA 298 to 314. Plasmid 654, TM deleted completely.
(b) The endogenous transmembrane domain can only be partially substituted for by foreign TM anchors.
Plasmid #513, HLA Cw3 full length. TM/IC exchange mutants are based on #693. Plasmid #739, sequences coding for post-translational GPI-link modification. Plasmid #740, complete TM portion of CD47. Plasmid #741, TM portion of H-2IA$\beta$ chain. Groups of four DBA/2 mice were electrovaccinated with 50 $\mu$g/animal of plasmid and blood samples were analyzed 13 days after electrovaccination.

Example 6

Reconstitution of highly effective minimal constructs

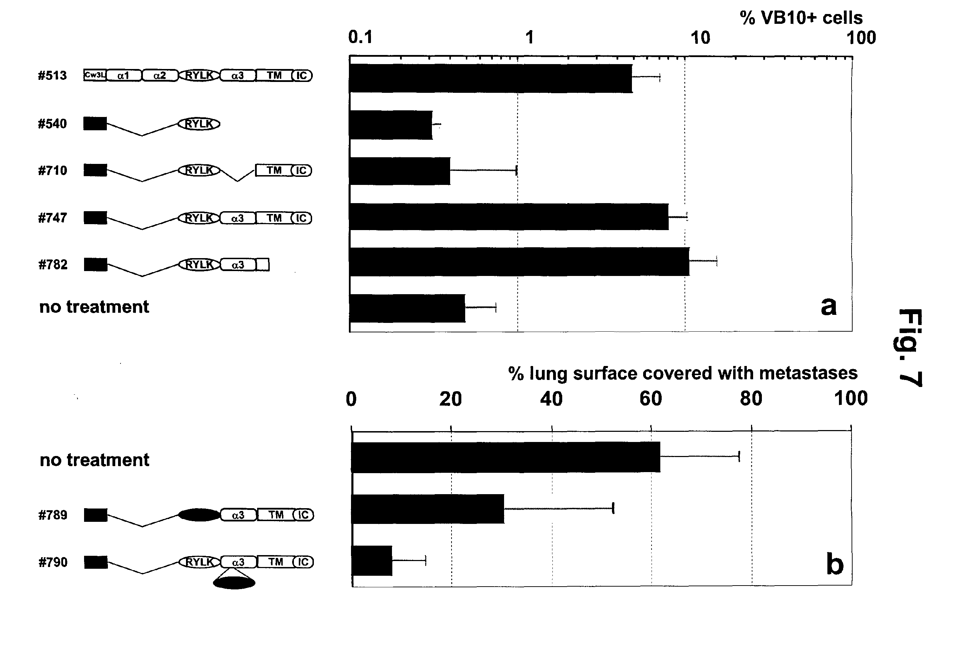

Finally, only those structural elements of HLA-Cw3 which were found to contribute to high immunogenicity were assembled. Plasmids were progressively refined and tested in vivo: Plasmid #540 carries the RYLK epitope preceded by the adenoviral leader E3. This construct was fused to the TM domain of HLA-Cw3 to create plasmid #710. Finally in plasmid #747 the endogenous $\alpha$3 domain was inserted between RYLK epitope and TM/IC domains. While plasmids #540 and #710 induced only low levels of VB10+CD8+ T cells, construct #747 performed better than the positive control #513 (Figure 7). In a further step the TM-domain of plasmid #747 was truncated to a rudimentary anchor of 16AA. As already shown for plasmid #709, this short anchor is sufficient for high in vivo immunogenicity of plasmid #782.

To test whether the newly defined efficient minimal constructs could be used to boost immunogenicity of CTL epitopes other than RYLK, tumor protection experiments on the B16 melanoma model were performed. The spontaneously arising murine melanoma B16 of C57BL/6 mice became a reference where antigen-specific vaccination against tumors can be tested (20, 27). Similar to human cancers, B16 cells are very weakly immunogenic, and sublines exist with varying tumorigenic and metastatic capabilities (28, 29). Murine tyrosinase-related protein (TRP)-2 has been identified as dominant tumor antigen upon expression cloning and screening with B16-reactive cytotoxic T cells. The peptide SVYDFFVWL was defined as the dominant H-2Kb-restricted epitope, and was shown to be of therapeutic benefit in the treatment of B16 lung metastasis (20, 30). For the experiments constructs harboring the SVYD-epitope were tested for potential therapeutic effects. Two plasmid variants based on the Cw3 minimal construct #747 were generated. In plasmid #789 the RYLK epitope was exchanged for the SVYD epitope and in plasmid #790 the SVYD epitope was mutagenized into a moderately homologous hydrophobic region of the $\alpha$3 domain. This was done to preserve the structure of the molecule and to prevent the signal recognition machinery from destroying the antigenic epitope. Since hydrophobic leader signals must be followed by small and neutral residues for correct cleavage by the signal peptidase (31), direct fusion of a highly hydrophobic epitope like SVYD to a signal sequence holds the potential risk of faulty cleavage inside or at the C-terminal side of the antigenic epitope.

Anti-tumor protection was tested in the B16 lung metastasis model, where previous attempts to induce tumor protection with full-length mTRP-2 electrovaccinations had failed (manuscript in preparation). Groups of C57/BL6 mice were electrovaccinated three times with intervals of one week, using plasmids #789 and #790. Control mice were left untreated. One week after the last vaccination mice were challenged by i.v. injection of $8\times10^5$ B16F10M melanoma cells.

On day 22 post-challenge, mice were sacrificed and lungs were examined for coverage with metastases. Both constructs conferred significant protection to vaccinated animals (Figure 7b), especially plasmid #790 causing a more than 7-fold decrease in coverage of lung surface with metastases.

Figure 7:

Newly defined, efficient minimal constructs can be utilized as carrier for an unrelated, weakly immunogenic CTL epitope.

(a) Four generations of progressively refined minimal constructs.
Groups of four DBA/2 mice were electrovaccinated with 50 μg/animal of plasmid and blood samples were analyzed 13 days after electrovaccination. Plasmid #513, HLA Cw3 full length. Plasmid #540, RYLK epitope preceded by Adenoviral E3 19k leader. Plasmid #710, #540 with endogenous TM domain fused. Plasmid #747, #540 with endogenous α3/TM domains fused. Plasmid #782, TM domain of #747 truncated to rudimentary 16 AA.

(b) Strong anti-tumor protection in the B16 lung metastasis model conferred by optimized minimal constructs.
Groups of DBA/2 mice with 10 animals each were electrovaccinated three times with intervals of one week, using 50 μg of plasmids #789 and #790; control mice were left untreated. One week after the last vaccination mice were challenged by i.v. injection of 8x10^5 B16F10M melanoma cells. Mice were sacrificed on day 22 post-challenge, and melanoma nodules were scored as percent coverage of lung surface. Compared to the untreated group, electrovaccination results in significant protection at the level of p<0.003 for plasmid #789, and p<0.001 for plasmid #790 (T-test). Plasmid #789, RYLK substituted by SVYD. Plasmid #790, SVYD inserted into α3 domain. Both constructs are based on #747. Note that Cw3-derived sequences are depicted with symbols on white background, whereas structural parts of various murine origin are on black background.

References

1. Stasney, J., Cantarow, A., Paschkis, K. E. 1950. Production of neoplasms by fractions of mammalian neoplasms. Cancer Res. 10:775.
2. Ito, Y. 1960. A tumor-producing factor extracted by phenol from papillomatous tissue (Shope) of cottontail rabbits. Virology 12:596.
3. Atanasiu, P. 1962. Production de tumerus chez le hamster par inoculation d'acide desoxyribonucleique extrait de cultures des tissues infectés par le virus de polyome. C.R.Acad.Sci.Paris 254:4228.
4. Wolff, J. A., R. W. Malone, P. Williams, W. Chong, G. Acsadi, A. Jani, and P. L. Felgner. 1990. Direct gene transfer into mouse muscle in vivo. Science 247:1465.
5. Yang, N. S., J. Burkholder, B. Roberts, B. Martinell, and D. McCabe. 1990. In vivo and in vitro gene transfer to mammalian somatic cells by particle bombardment. Proc. Natl. Acad. Sci. U. S. A. 87:9568.
6. Tang, D. C., M. DeVit, and S. A. Johnston. 1992. Genetic immunization is a simple method for eliciting an immune response. Nature 356:152.
7. Ulmer, J. B., J. J. Donnelly, S. E. Parker, G. H. Rhode, P. L. Felgner, V. J. Dwarki, S. H. Gromowski, R. R. Deck, C. M. DeWitt, A. Friedman, L. A. Hawe, K. R. Leander, D. Martinez, H. C. Perry, J. E. Shiver, D. L. Montgomery, and M. A. Liu. 1993. Heterologous protection against influenza by injection of DNA encoding a viral protein. Science 259:1745.
8. Eisenbraun, M. D., D. H. Fuller, and J. R. Haynes. 1993. Examination of parameters affecting the elicitation of humoral immune responses by particle bombardment-mediated genetic immunization. DNA Cell Biol. 12:791.
9. Wang, B., J. Boyer, V. Srikantan, L. Coney, R. Carrano, C. Phan, M. Merva, K. Dang, M. Agadjanan, L. Gilbert, and et al. 1993. DNA inoculation induces neutralizing immune responses against human immunodeficiency virus type 1 in mice and nonhuman primates. DNA Cell Biol. 12:799.
10. Wang, R., D. L. Doolan, T. P. Le, R. C. Hedstrom, K. M. Coonan, Y. Charoenvit, T. R. Jones, P. Hobart, M. Margalith, J. Ng, W. R. Weiss, M. Sedegah, C. de Taisne, J. A. Norman, and S. L. Hoffman. 1998. Induction of antigen-specific cytotoxic T lymphocytes in humans by a malaria DNA vaccine. Science 282:476.
11. Skipper, J. C. A., R. C. Hendrickson, P. H. Gulden, V. Brichard, A. Van Pel, Y. Chen, J. Shabanowitz, T. Wolfel, C. L. J. Slingluff, T. Boon, D. F. Hunt, and V. H. Engelhard. 1996. An HLA-A2-restricted tyrosinase antigen on melanoma cells results from posttranslational modification and suggests a novel pathway for processing of membrane proteins. J. Exp. Med. 183:527.
12. Ossendorp, F., M. Eggers, A. Neisig, T. Ruppert, M. Groetrupp, A. Sijts, E. Mengede, P. Kloetzel, J. Neefjes, U. Koszinowski, and C. Melief. 1996. A single residue exchange within a viral CTL epitope alters proteasome-

mediated degradation resulting in lack of antigen presentation. Immunity 5:115.

13. Fu, T. M., L. M. Mylin, T. D. Schell, I. Bacik, G. Russ, J. W. Yewdell, J. R. Bennink, and S. S. Tevethia. 1998. An endoplasmic reticulum-targeting signal sequence enhances the immunogenicity of an immunorecessive simian virus 40 large T antigen cytotoxic T-lymphocyte epitope. J.Virol. 72:1469.

14. Restifo, N. P., I. Bacik, K. R. Irvine, J. W. Yewdell, B. J. McCabe, R. W. Anderson, L. C. Eisenlohr, S. A. Rosenberg, and J. R. Bennink. 1995. Antigen processing in vivo and the elicitation of primary CTL responses. J. Immunol. 154:4414.

15. Maryanski, J. L., R. S. Accolla, and B. Jordan. 1986. H2-restricted recognition of cloned HLA class I gene products expressed in mouse cells. J Immunol 136:4340.

16. Casanova, J. L., J. C. Cerottini, M. Matthes, A. Necker, H. Gournier, C. Barra, C. Widmann, H. R. MacDonald, F. Lemonnier, B. Malissen, and J. L. Maryanski. 1992. H-2-restricted cytolytic T lymphocytes specific for HLA display T cell receptors of limited diversity. J. Exp. Med. 176:439.

17. MacDonald, H. R., J. L. Casanova, J. L. Maryanski, and J. C. Cerottini. 1993. Oligoclonal expansion of major histocompatibility complex class I- restricted cytolytic T lymphocytes during a primary immune response in vivo: direct monitoring by flow cytometry and polymerase chain reaction. J. Exp. Med. 177:1487.

18. Bousso, P., A. Casrouge, J. D. Altman, M. Haury, J. Kanellopoulos, J. P. Abastado, and P. Kourilsky. 1998. Individual variations in the murine T cell response to a specific peptide reflect variability in naive repertoires. Immunity 9:169.

19. Widera, G., M. Austin, D. Rabussay, C. Goldbeck, S. W. Barnett, M. Chen, L. Leung, G. R. Otten, K. Thudium, M. J. Selby, and J. B. Ulmer. 2000. Increased DNA vaccine delivery and immunogenicity by electroporation in vivo. J.Immunol. 164:4635.

20. Bloom, M. B., D. Perry-Lalley, P. F. Robbins, Y. Li, M. el-Gamil, S. A. Rosenberg, and J. C. Yang. 1997. Identification of tyrosinase-related protein 2 as a tumor rejection antigen for the B16 melanoma. J. Exp. Med. 185:453.

21. Sato, Y., M. Roman, H. Tighe, D. Lee, M. Corr, M.-D. Nguyen, G. J. Silverman, M. Lotz, D. A. Carson, and E. Raz. 1996. Immunostimulatory DNA sequences necessary for effective intradermal gene immunization. Science 273:352.

22. Attuil, V., P. Bucher, M. Rossi, M. Mutin, and J. L. Maryanski. 2000. Comparative T cell receptor repertoire selection by antigen after adoptive transfer: a glimpse at an antigen-specific preimmune repertoire. Proc.Natl.Acad. Sci.U.S.A 97:8473.

23. Walker, P. R., T. Ohteki, J. A. Lopez, H. R. MacDonald, and J. L. Maryanski. 1995. Distinct phenotypes of antigen-selected CD8 T cells emerge at different stages of an in vivo immune response. J.Immunol. 155:3443.

24. Bacik, I., J. H. Cox, R. Anderson, J. W. Yewdell, and J. R. Bennink. 1994. TAP (transporter associated with antigen processing)-independent presentation of endogenously synthesized peptides is enhanced by endoplasmic reticulum insertion sequences located at the amino-but not carboxyl-terminus of the peptide. J.Immunol. 152:381.

25. Livingston, B. D., C. Crimi, H. Grey, G. Ishioka, F. V. Chisari, J. Fikes, R. W. Chesnut, and A. Sette. 1997. The hepatitis B virus-specific CTL responses induced in humans by lipopeptide vaccination are comparable to those elicited by acute viral infection. J.Immunol. 159:1383.

26. Gerloni, M., S. Xiong, S. Mukerjee, S. P. Schoenberger, M. Croft, and M. Zanetti. 2000. Functional cooperation between T helper cell determinants. Proc.Natl.Acad.Sci.U.S.A. 97:13269.

27. Jaffee, E. M., and D. M. Pardoll. 1996. Murine tumor antigens: is it worth the search? Curr. Opin. Immunol. 8:622.

28. Zhu, D. Z., C. F. Cheng, and B. U. Pauli. 1991. Mediation of lung metastasis of murine melanomas by a lung-specific endothelial cell adhesion molecule. Proc. Natl. Acad. Sci. U. S. A. 88:9568.

29. Kobayashi, T., K. Urabe, A. Winder, C. Jimenez-Cervantes, G. Imokawa, T. Brewington, F. Solano, J. C. Garcia-Borron, and V. J. Hearing. 1994. Tyrosinase related protein 1 (TRP1) functions as a DHICA oxidase in melanin biosynthesis. EMBO J. 13:5818.

30. Schreurs, M. W., A. A. Eggert, A. J. de Boer, J. L. Vissers, T. van Hall, R. Offringa, C. G. Figdor, and G. J. Adema. 2000. Dendritic cells break tolerance and induce protective immunity against a melanocyte differentiation antigen in an autologous melanoma model. Cancer Res. 60:6995.

31. Stroud, R. M., and P. Walter. 1999. Signal sequence recognition and protein targeting. Curr.Opin.Struct.Biol. 9:754.

32. Ciernik, I. F., J. A. Berzofsky, and D. P. Carbone. 1996. Induction of cytotoxic T lymphocytes and antitumor immunity with DNA vaccines expressing single T cell epitopes. J.Immunol. 156:2369.

33. Corr, M., D. J. Lee, D. A. Carson, and H. Tighe. 1996. Gene vaccination with naked plasmid DNA: mechanism of CTL priming. J. Exp. Med. 184:1555.

34. Iwasaki, A., C. A. Torres, P. S. Ohashi, H. L. Robinson, and B. H. Barber. 1997. The dominant role of bone marrow-derived cells in CTL induction following plasmid DNA immunization at different sites. J.Immunol. 159:11.

35. Muniz, M., and H. Riezman. 2000. Intracellular transport of GPI-anchored proteins. EMBO J. 19:10.

36. Gromme, M., F. G. Uytdehaag, H. Janssen, J. Calafat, R. S. van Binnendijk, M. J. Kenter, A. Tulp, D. Verwoerd, and J. Neefjes. 1999. Recycling MHC class I molecules and endosomal peptide loading. Proc.Natt.Acad.Sci.U.S.A 96:10326.

37. Rescigno, M., and P. Borrow. 2001. The host-pathogen interaction: new themes from dendritic cell biology. Cell 106:267.

Bjorkman, P. J., Saper M. A., Samraoui B., Bennett W. S., Strominger J. L., and Wiley D. C.. Structure of the human class I histocompatibility antigen, HLA-A2. *Nature* 329: 506-512, 1987.

Blomberg, K. und Ulfstedt, A.C., 1993, J.lmmunol.Methods 160:27-34

Boisgerault, F., Khalil I., Tieng V., Connan F., Tabary T., Cohen J. H., Choppin J., Charron D., and Toubert A.. Definition of the HLA-A29 peptide ligand motif allows prediction of potential T-cell epitopes from the retinal soluble antigen, a candidate autoantigen in birdshot retinopathy. *Proc.Natl.Acad.Sci.U.S.A.* 93 (8):3466-3470, 1996.

Coligan, J.E., et al., 1991, *Current Prot. in Immunol.,* Wiley, New York

Coulie, 1997, *Mol. Med. Today* **3**:261-268

Etz, H., Duc Bui Minh, Henics T., Dryla A., Winkler B., Triska Ch., Boyd A.P., Sollner J., Schmidt W., von Ahsen U., Buschle M., Gill S.R., Kolonay J., Khalak H., Fraser C.M., von Gabain A., Nagy E., and Meinke A.. Identification of in vivo expressed vaccine candidate antigens from Staphylococcus aureus. *PNAS* 99 (10):6573-6578, 2002.

Feltkamp, M.C. et al., 1995, *Eur. J. Immunol.* 25 (9), 2638-2642

Fidler, et al., (1975), *Cancer Res.* 35: 218-234

Grohmann, U., et al., 1995, *Eur. J. Immunol.* 25, 2797-2802

Hanke, T., Samuel R. V., Blanchard T. J., Neumann V. C., Allen T. M., Boyson J. E., Sharpe S. A., Cook N., Smith G. L., Watkins D. I., Cranage M. P., and McMichael A. J.. Effective induction of simian immunodeficiency virus-specific cytotoxic T lymphocytes in macaques by using a multiepitope gene and DNA prime-modified vaccinia virus Ankara boost vaccination regimen. *J.Virol.* 73 (9):7524-7532, 1999.

Hodge, J. W.. Carcinoembryonic antigen as a target for cancer vaccines. *Cancer Immunol.Immunother.* 43 (3):127-134, 1996.

Kawakami, Y., Eliyahu S., Delgado C. H., Robbins P. F., Sakaguchi K., Appella E., Yanelli J. R., Adema G. J., Miki T., and Rosenberg S. A.. Identification of a human melanoma antigen recognized by tumor-infiltrating lymphocytes associated with in vivo tumor rejection. *Proc.Natl.Acad.Sci.USA* 91: 6458-6462, 1994.

Konopitzky, R., König U., Meyer R. G., Sommergruber W., Wölfel T., and Schweighoffer T.. Identification of HLA-A*0201-Restricted T Cell Epitopes Derived From the Novel Overexpressed Tumor Antigen Calcium-Activated Chloride Channel 2. *J.Immunol.* 169 (1):540-547, 2002.

Leong, J. M., Fournier, R. S., and Isberg, R. R.. Identification of the integrin binding domain of the Yersinia pseudotuberculosis invasin protein. *EMBO J.* 9:1979-1989, 1990.

Maddon, P. J., Littman D. R., Godfrey M., Maddon D. E., Chess L., and Axel R. The isolation and nucleotide sequence of a cDNA encoding the T cell surface protein T4: A new member of the immunoglobulin gene family. *Cell* 42: 93-104, 1985.

Migaki, G. I., Kahn J., and Kishimoto T. K.. Mutational analysis of the membrane-proximal cleavage site of L-selectin: relaxed sequence specificity surrounding the cleavage site. *J.Exp.Med.* 182 (2):549-557, 1995.

Mizushima, S. and Nagata, S.. pEF-BOS, a powerful mammalian expression vector. *Nucleic.Acids.Res.* 18 (17):5322-5322, 1990.

Puccetti, P. et al., 1995, *Eur. J. Immunol.* 24, 1446-1452

Remington's Pharmaceutical Sciences, Gennaro AR ed. 20th edition, 2000: Williams & Wilkins PA, USA

Robbins, P. F. and Kawakami, Y.. Human tumor antigens recognized by T cells. *Curr.Opin.Immunol.* 8:628-636, 1996.

Rodolfo, M., Zilocchi C., Melani C., Cappetti B., Arioli I., Parmiani G., and Colombo M. P.. Immunotherapy of experimental metastases by vaccination with interleukin gene-transduced adenocarcinoma cells sharing tumor- associated antigens. Comparison between IL-12 and IL-2 gene- transduced tumor cell vaccines. *J.Immunol.* 157 (12): 5536-5542, 1996.

Schweighoffer, T.. Molecular cancer vaccines: tumor therapy using antigen-specific immunizations. *Path.Onc. Res.* 3 (3):164-176, 1997.

Simmons, D. L. Cloning cell surface molecules by transient expression in mammalian cells. In: *Cellular interactions in development. A practical approach,* edited by D. A. Hartley, Oxford:IRL Press, 1993, p. 93-127.

Steinman, L. Multiple sclerosis: a coordinated immunological attack against myelin in the central nervous system. *Cell* 85 (3):299-302, 1996.

Sullivan NJ, Sanchez A, Rollin PE, Yang ZY, and Nabel GJ. Development of a preventive vaccine for Ebola virus infection in primates. *Nature.* 408 (6812):605-609, 2000.

Sykes, K. F. and Johnston, S. A.. Linear expression elements: a rapid, in vivo, method to screen for gene functions. *Nat.Biotechnol.* 17 (4):355-359, 1999.

Takahashi K, Vigneron M, Matthes H, Wildeman A, Zenke M, Chambon P., Requirement of stereospecific alignments for initiation from the simian virus 40 early promoter. *Nature.* 1986 Jan 9-15;319(6049):121-6.

Tuting, 1997, Eur. *J. Immunol.* 27:1702-2707

Vitiello, A. et al, 1995, *J. Clin. Inv.* 95, 1, 341-349

Wang, R. F., Appella E., Kawakami Y., Kang X., and Rosenberg S. A.. Identification of TRP-2 as a human tumor antigen recognized by cytotoxic T lymphocytes. *J.Exp.Med.* 184:2207-2216, 1996.

Widmann, C. et al., 1992, *J. Immunol. Methods* 155 (1), 95-99

Yang, B., Hahn Y. S., Hahn C. S., and Braciale T. J.. The requirement for proteasome activity in class I major histocompatibility complex antigen presentation is dictated by the length of preprocessed antigen. *J.Exp.Med.* 183: 1545-1552, 1996.

Zucchelli S., Capone S., Fattori E., Folgori A., Di Marco A., Casimiro D., Simon A.J., Laufer R., La Monica N., Cortese R., and Nicosia A., Enhancing B- and T-Cell Immune Response to a Hepatitis C Virus E2 DNA Vaccine by Intramuscular Electrical Gene Transfer. *J.Virol.* 74 (24):11598-11607, 2000.

**Claims**

1. A recombinant DNA molecule containing a sequence selected from

   5'-L-(AG)*n*-A3-TM- 3',

   5'-L-A3.1-(AG)*n*-A3.2-TM- 3', and

   5'-L-A3-(AG)*n*-TM-3', wherein

   L denotes a leader sequence encoding a signal peptide that ensures delivery of the expressed molecule into the endoplasmic reticulum (ER) and cleavage of the signal peptide from the molecule; wherein

   AG denotes a sequence encoding an antigen or an antigen fragment or an antigenic peptide; wherein

   A3 denotes a sequence encoding the $\alpha 3$ region, or a section thereof, of an HLA heavy chain; wherein

   TM denotes a sequence encoding a transmembrane region, or a section thereof, of a membrane spanning protein, and wherein $n \geq 1$.

2. The recombinant DNA molecule of claim 1, wherein L encodes a signal peptide that comprises or is derived from the signal peptide of a eukaryotic protein.

3. The recombinant DNA molecule of claim 2, wherein the eukaryotic protein is a transmembrane protein.

4. The recombinant DNA molecule of claim 3, wherein the transmembrane protein is a type I transmembrane protein

5. The recombinant DNA molecule of claim 4, wherein the type I transmembrane protein is selected from the group of CD4, CD8 and membrane-bound B cell receptor.

6. The recombinant DNA molecule of claim 2, wherein L encodes a signal pepide that comprises or is derived from a signal peptide of a secreted protein.

7. The recombinant DNA molecule of claim 6, wherein the secreted protein is an immunoglobulin.

8. The recombinant DNA molecule of claim 3, wherein the protein is an HLA molecule.

9. The recombinant DNA molecule of claim 8, wherein the HLA molecule is H LA-Cw3.

10. The recombinant DNA molecule of claim 8, wherein the HLA molecule is HLA-A*0201.

11. The recombinant DNA molecule of claim 2, wherein L encodes a signal peptide that comprises or is derived from the signal peptide of a viral protein.

12. The recombinant DNA molecule of claim 11, wherein the viral protein is adenovirus E3 19K.

13. The recombinant DNA molecule of claim 11, wherein the viral protein is influenza hemagglutinin (HA).

14. The recombinant DNA molecule of claim 11, wherein the viral protein is hepatitis B virus surface antigen (HbsAg).

15. The recombinant DNA molecule of claim 2, wherein AG encodes a (poly)peptide that comprises or is derived from a protein of a bacterial pathogen.

16. The recombinant DNA molecule of claim 2, wherein AG encodes a (poly)peptide that comprises or is derived from a protein of a viral pathogen.

17. The recombinant DNA molecule of claim 2, wherein AG encodes a (poly)peptide that comprises or is derived from a human tumor antigen.

18. The recombinant DNA molecule of claim 2, wherein AG encodes an autoantigen or a protein fragment or peptide derived from it.

19. The recombinant DNA molecule of any one of claims 15 to 18, wherein n>1 and wherein AG encodes identical or different antigen peptides.

20. The recombinant DNA molecule of claim 19, wherein n= 2 - ca. 30.

21. The recombinant DNA molecule of claim 2, wherein A3 encodes the $\alpha 3$ sequence, or a portion thereof, of HLA Class I molecule heavy chain.

22. The recombinant DNA molecule of claim 21, wherein the HLA Class I molecule is HLA-Cw3.

23. The recombinant DNA molecule of claim 21, wherein the HLA Class I molecule is HLA-A*0201.

24. The recombinant DNA molecule of any one of claims 20 to 23, wherein A3 encodes the full-length $\alpha 3$ domain.

25. The recombinant DNA molecule of any one of claims 15 to 24, wherein the AG sequence is inserted in the A3 sequence.

26. The recombinant DNA molecule of claim 1, wherein TM encodes a transmembrane region that comprises or is derived from the transmembrane region of a eukaryotic transmembrane protein.

27. The recombinant DNA molecule of claim 26, wherein the transmembrane protein is a type I transmembrane protein.

28. The recombinant DNA molecule of claim 27, wherein the type I transmembrane protein is selected from the group of CD4, CD8 and membrane-bound B cell receptor.

29. The recombinant DNA molecule of claim 1, wherein TM encodes a transmembrane region that comprises or is derived from the transmembrane region of a secreted eukaryotic protein.

30. The recombinant DNA molecule of claim 29, wherein the protein is a transmembrane form of an immunoglobulin.

31. The recombinant DNA molecule of claim 26, wherein the transmembrane protein is an HLA molecule.

32. The recombinant DNA molecule of claim 31, wherein the HLA molecule is HLA-Cw3.

33. The recombinant DNA molecule of claim 31, wherein the HLA molecule is HLA-A*0201.

34. The recombinant DNA molecule of any one of claims 26 to 33, wherein the TM sequence is extended at the 5' or the 3'end.

35. The recombinant DNA molecule of claim 34, wherein the TM sequence is extended by a sequence encoding a reporter molecule.

36. The recombinant DNA molecule of claim 34, wherein the TM sequence is extended by a tag.

37. The recombinant DNA molecule of claim 1, wherein the L sequence, the A3 sequence and the TM sequence are all derived from HLA-Cw3.

**38.** The recombinant DNA molecule of claim 1 in the form of a vector that carries the regulatory sequences required for expression in a human cell.

**39.** The recombinant DNA molecule of claims 1 and 38 in the form of a plasmid that is capable of self-replication in bacteria.

**40.** A pharmaceutical composition, containing, as the active ingredient, a recombinant DNA molecule of any of claims 1 to 39.

**41.** The pharmaceutical composition of claims 40, wherein the recombinant DNA molecule is inserted in a viral vector.

Fig. 1

# Fig. 2

# Fig. 3

b

pg/ml mIFNγ

300

200

100

0

a

% VB10+ cells

100

10

1

0.1

#513 — Cw3L α1 α2 RYLK α3 TM IC

#653 — Cw3L α1 α2 RYLK α3

#654 — Cw3L α1 α2 RYLK

#655 — Cw3L α1

**Fig. 4**

Fig. 5

# Fig. 6

Fig. 7

**European Patent Office**

## EUROPEAN SEARCH REPORT

Application Number

EP 02 01 9592

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 5 817 315 A (ATKIN ANDREW) 6 October 1998 (1998-10-06) se whole doc. esp. claims and col.3, l.1-15 | 1-40 | C07K14/705 |
| X | WO 94 25610 A (TYKOCINSKI MARK L) 10 November 1994 (1994-11-10) see whole doc. esp. claims and p.4,l.15-19, p.5,l.4-8 and p.8,l.3-11 and p.25,l.20 ff. | 1-40 | |
| A | FAYEN J ET AL: "SOLUBLE HUMAN CD8ALPHA BINDS TO A RECOMBINANT FUSION PROTEIN WHICH INCORPORATES THE CLASS I MHC ALPHA3 DOMAIN" JOURNAL OF IMMUNOLOGY, THE WILLIAMS AND WILKINS CO. BALTIMORE, US, vol. 150, no. 8, PART 2, 15 April 1993 (1993-04-15), page 288A XP002018446 ISSN: 0022-1767 * the whole document * | | |
| A | WO 99 13095 A (GRETEN TIM ;HERRIN SEAN M O (US); PARDOLL DREW (US); SLANSKY JILL) 18 March 1999 (1999-03-18) * the whole document * | | **TECHNICAL FIELDS SEARCHED (Int.Cl.7)** C07K |
| T | PASTER W. ET AL., : "structural elements of a protein antigen determine immunogenicity of the embedded mhc class I-restricted T cell epitope" J IMMUNOLOGY, vol. 169, no. 6, 15 September 2002 (2002-09-15), pages 2937-2946, XP002229695 * the whole document * | | |

The present search report has been drawn up for all claims

| Place of search | Date of completion of the search | Examiner |
|---|---|---|
| MUNICH | 3 February 2003 | Mueller, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 1 394 181 A1**

## ANNEX TO THE EUROPEAN SEARCH REPORT
## ON EUROPEAN PATENT APPLICATION NO.

EP 02 01 9592

This annex lists the patent family members relating to the patent documents cited in the above-mentioned European search report.
The members are as contained in the European Patent Office EDP file on
The European Patent Office is in no way liable for these particulars which are merely given for the purpose of information.

03-02-2003

| Patent document cited in search report | | Publication date | Patent family member(s) | | Publication date |
|---|---|---|---|---|---|
| US 5817315 | A | 06-10-1998 | AU | 6715094 A | 21-11-1994 |
| | | | WO | 9425054 A1 | 10-11-1994 |
| WO 9425610 | A | 10-11-1994 | AU | 6908094 A | 21-11-1994 |
| | | | WO | 9425610 A1 | 10-11-1994 |
| WO 9913095 | A | 18-03-1999 | AU | 9477698 A | 29-03-1999 |
| | | | CA | 2303396 A1 | 18-03-1999 |
| | | | EP | 1012320 A2 | 28-06-2000 |
| | | | JP | 2001515726 T | 25-09-2001 |
| | | | WO | 9913095 A2 | 18-03-1999 |
| | | | US | 6268411 B1 | 31-07-2001 |
| | | | US | 2002006903 A1 | 17-01-2002 |